# EUROPÄISCHE PATENTSCHRIFT

(11) **EP 1 063 983 B1**
(45) Veröffentlichungstag und Bekanntmachung des Hinweises auf die Patenterteilung: **05.12.2001**
(21) Anmeldenummer: 99913259.0
(22) Anmeldetag: 16.03.1999
(51) Int. Cl.: A61K 31/215, A61K 31/135, A61K 9/70, A61L 15/44

(54) **TRANSDERMALES THERAPEUTISCHES SYSTEM (TTS) OXYBUTYNIN ENTHALTEND**
TRANSDERMAL THERAPEUTIC SYSTEM (TTS) CONTAINING OXYBUTYNIN
SYSTEME THERAPEUTIQUE TRANSDERMIQUE (TTS) CONTENANT DE L'OXYBUTYNINE

(30) Priorität: 20.03.1998 DE 19812413
(43) Veröffentlichungstag der Anmeldung: 03.01.2001
(73) Patentinhaber: SCHWARZ PHARMA AG, 40789 Monheim (DE)
(72) Erfinder: ARTH, Christoph, D-40593 Düsseldorf (DE); KOLLMEYER-SEEGER, Andreas, D-40764 Langenfeld (DE); RIMPLER, Stephan, D-40723 Hilden (DE); WOLFF, Hans-Michael, D-40789 Monheim (DE)
(86) Internationale Anmeldenummer: EP9901707
(87) Internationale Veröffentlichungsnummer: WO9948493

(56) Entgegenhaltungen:
- WO-A-95/09007
- WO-A-96/33678
- DE-A- 4 310 012
- DATABASE WPI Section Ch, Week 9244 Derwent Publications Ltd., London, GB; Class A96, AN 92-363072 XP002900529 & JP 04 266821 A (RIDO CHEM KK), 22. September 1992 (1992-09-22)

## Beschreibung

Die vorliegende Erfindung betrifft ein Transdermales Therapeutisches System (TTS) zur transcutanen Verabreichung von Oxybutynin über mehrere Tage sowie ein Verfahren zu seiner Herstellung ohne Verwendung von Lösungsmitteln.

Die Bioverfügbarkeit von oral oder intravenös verabreichten Wirkstoffen ist oft unbefriedigend. Die hepatische Metabolisierung vieler Wirkstoffe kann bei der ersten Leberpassage zu unerwünschten Konzentrationsverhältnissen, toxischen Nebenprodukten und zur Verminderung der Wirkung oder gar zum Wirkverlust führen. Gegenüber oraler Verabreichung besitzt die transdermale Gabe von Wirkstoffen verschiedene Vorteile. Die Wirkstoffzufuhr läßt sich über einen längeren Zeitraum besser steuern, wodurch hohe Blutspiegelschwankungen vermieden werden. Zudem kann die erforderliche therapeutisch wirksame Dosis meist deutlich verringert werden. Außerdem wird ein Pflaster vom Patienten oft mehr bevorzugt als täglich einmal oder mehrfach einzunehmende Tabletten.

In der Vergangenheit wurde zur Überwindung der vorgenannten Nachteile der nichttransdermalen Gabe von Wirkstoffen durch eine Vielzahl von Transdermalen Therapeutischen Systemen (TTS) mit unterschiedlichem Aufbau für verschiedene Wirkstoffe zur Therapie unterschiedlicher Erkrankungen Rechnung getragen.

So beschreiben die nachfolgend genannten technischen Dokumente für eine breite Vielfalt systemisch oder lokal reagierender Wirkstoffe deren parenterale Verabreichung entweder auf Basis dosis-kontrollierender oder allgemein freisetzender Systeme. Beispielhaft sind dies: U.S.P.
3,598,122; 3,598,123; 3,731,683; 3,797,494; 4,031,894; 4,201,211; 4,286,592; 4,314,557; 4,379,454; 4,435,180; 4,559,222; 4,568,343; 4,573,995, 4,588,580; 4,645,502; 4,702,282; 4,788,062; 4,816,258; 4,849,226; 4,908,027; 4,943,435 und 5,004,610.

In den späten sechziger Jahren dieses Jahrhunderts war ursprünglich theoretisch angenommen worden, daß jeder Wirkstoff mit kurzer Halbwertszeit aber hoher Wirksamkeit und guter Hautdurchgängigkeit für eine sichere und effektive Verabreichung mittels eines TTS geeignet sei. Diese anfänglichen Erwartungen hinsichtlich der Möglichkeiten der transdermalen Verabreichung von Wirkstoffen mittels TTS konnten jedoch nicht erfüllt werden. Dies findet seine Begründung hauptsächlich darin, daß die Haut von Natur aus mit einer unüberschaubaren Vielfalt von Eigenschaften ausgestattet ist, um ihre Funktion als intakte Barriere gegenüber dem Eindringen von nicht-körpereigenen Substanzen in den Körper aufrecht zu erhalten. (Siehe hierzu: Transdermal Drug Delivery: Problems and Possibilities, B.M. Knepp et al., CRC Critical Review and Therapeutic Drug Carrier Systems, Vol. 4, Issue 1 (1987)).

Daher steht die transdermale Verabreichung nur für diejenigen wenigen Wirkstoffe zur Verfügung, die eine geeignete Kombination von vielen günstigen Charakteristika aufweisen. Für einen bestimmten Wirkstoff sind diese geforderten Charakteristika, die die sichere und effektive transdermale Verabreichung gewährleisten sollen, jedoch nicht vorhersagbar.

Die an einen für die transdermale Verabreichung geeigneten Wirkstoff zu stellenden Anforderungen sind:
- Hautdurchgängigkeit,
- keine Beeinträchtigung des Klebevermögens des Pflasters durch den Wirkstoff,
- Vermeidung von Hautirritationen,
- Vermeidung von allergischen Reaktionen,
- günstige pharmakokinetische Eigenschaften,
- günstige pharmakodynamische Eigenschaften,
- ein relativ weites therapeutisches Fenster,
- Metabolismuseigenschaften, die konsistent mit der therapeutischen Anwendung bei kontinuierlicher Gabe sind.

Unzweifelhaft ist die vorgenannte Liste der Anforderungen nicht erschöpfend. Damit ein Wirkstoff für die transdermale Verabreichung zur Verfügung stehen kann, ist die "richtige" Kombination all dieser Anforderungen wünschenswert.

Das für die Wirkstoffe vorgenannte gilt in gleicher Weise für die den jeweiligen Wirkstoff enthaltende TTS-Zusammensetzung und deren konstruktiven Aufbau.

Üblicherweise handelt es sich bei den Transdermalen Therapeutischen Systemen (TTS) um Pflaster, die mit einer undurchlässigen Deckschicht, einer abziehbaren Schutzschicht und einer wirkstoffhaltigen Matrix oder einem wirkstoffhaltigen Reservoir mit semipermeabler Membran ausgestattet sind. Im ersten Fall werden sie als Matrixpflaster, im zweiten Fall als Membransystem bezeichnet.

Für die Deckschicht werden üblicherweise Polyester, Polypropylen, Polyethylen, Polyurethan etc. verwendet, die auch metallisiert oder pigmentiert sein können. Für die abziehbare Schutzschicht kommen u.a. Polyester, Polypropylen oder auch Papier mit Silikon- und/oder Polyethylenbeschichtung in Betracht.

Für die pharmazeutisch bzw. medizinisch üblichen wirkstoffhaltigen Matrices werden Stoffe auf Basis von Polyacrylat, Silikon, Polyisobutylen, Butylkautschuk, Styrol/ Butadien-Copolymerisat oder Styrol/lsopren-Copolymerisat verwendet.

Die in Membransystemen verwendeten Membranen können mikroporös oder semipermeabel sein und werden üblicherweise auf Basis eines inerten Polymeren, insbesondere Polypropylen, Polyvinylacetat oder Silikon gebildet

Während die wirkstoffhaltigen Matrixzusammensetzungen selbstklebend sein können, ergeben sich aber auch in Abhängigkeit von eingesetztem Wirkstoff wirkstoffhaltige Matrices, die nicht selbstklebend sind, so daß als Folge hiervon das Pflaster oder TTS konstruktiv mit einem Overtape versehen werden muß.

Zur Sicherstellung der erforderlichen Fluxrate des Wirkstoffes sind häufig Hautpenetrationsenhancer wie aliphatische, cycloaliphatische und/oder aromatisch-aliphatische Alkohole, jeweils ein- oder mehrwertig und jeweils mit bis zu 8 C-Atomen umfassend, ein Alkohol-/Wasser-Gemisch, ein gesättigter und/oder ungesättigter Fettalkohol mit jeweils 8 bis 18 Kohlenstoffatomen, eine gesättigte und/oder ungesättigte Fettsäure mit jeweils 8 bis 18 Kohlenstoffatomen und/oder deren Ester sowie Vitamine als Zusatz erforderlich.

Weiterhin werden häufig Stabilisatoren, wie Polyvinylpyrrolidon, α-Tocopherolsuccinat, Propylgallat, Methionin, Cystein und/oder Cystein-hydrochlorid, der wirkstoffhaltigen Matrix zugesetzt.

Wie die vorgenannte Aufstellung zeigt, sind zahlreiche TTS-Konstruktionen und hierfür verwendete Materialien bekannt. Allerdings sind viele interagierende Erfordernisse zu berücksichtigen, wenn ein Medikament in Form eines TTS einem medizinischen Bedürfnis genügen soll.

Die folgenden Problemstellungen sind bei der Entwicklung von wirkstoffhaltigen TTS zu berücksichtigen:
1. Die Hautpermeabilität für den Wirkstoff ist zu niedrig, um die therapeutisch notwendige Penetrationsrate zu erzielen und/oder die Verzögerungszeit ("lag-time") bis zum Erreichen der therapeutisch erforderlichen Plasmaspiegel ist zu lang, mit der Folge, daß hautpenetrationsbeschleunigende Zusätze verabreicht werden müssen.
2. Die wirkstoffbeladene und ggf. zusätzlich mit Hautpenetrationsenhancem beladene Polymermatrix ist bei längerer Lagerung physikalisch nicht stabil. Insbesondere kann eine Wirkstoffrekristallisation auftreten, die zu einer nicht kontrollierbaren Abnahme der Wirkstofffreisetzungskapazität des TTS führt.
3. Eine hohe Beladung des polymeren Trägerstoffes mit Wirkstoff und/oder Hautpenetrationsenhancern erschwert bei selbstklebenden Polymerfilmen die Einstellung optimaler Hafteigenschaften des transdermalen Systems.
4. Die Wirkstoffresorptionsrate sinkt bei Anwendungen über mehrere Tage in nicht akzeptabler Weise ab, so daß zusätzliche Steuerschichten und/oder -komponenten erforderlich sind.
5. Werden wirkstoffbeladene Schichten aus organischen Lösungen hergestellt, tritt das Problem des Verbleibens von Lösemittelresten in der wirkstoffhaltigen Schicht nach dem Trocknungsprozeß auf. Zusätzlich besteht die Gefahr einer unerwünschten Verdunstung von flüchtigen Hilfsstoffen während der Herstellung. Da aus Gründen der physikalischen Stabilität und Hautverträglichkeit des Systems in der Regel vollständige Lösungsmittelfreiheit anzustreben ist, muß das Reservoir gegebenenfalls in mehreren Schichten aufgebaut werden. Dies wiederum führt zu einer Erhöhung der Herstellungskosten.
6. Ferner ist aus der Literatur bekannt, daß die zur Penetrationsförderung durch die Haut häufig eingesetzten Fettsäuresester mehrwertiger Alkohole schwankende Qualität und unreine Verschnittmittel aufweisen. Dies führt zu schlecht reproduzieraren Penetrationsteigerungen (Burkoth et al. 1996, **DE 196 22 902**).

Die beschriebenen Probleme bedingen daher eine Vielzahl von Ausführungsformen Transdermaler Therapeutischer Systeme, die sich im Stand der Technik auf diesem Gebiet widerspiegeln.

Eine neuere Übersicht hierzu gibt beispielsweise **U.S. P 5,662,926** (Wick et al., 1997). Dieses Dokument beschreibt transdermale Systeme, die einen monolithischen, thermoplastischen Polymerfilm enthalten, in dem ein Wirkstoff, vorzugsweise Nikotin, homogen verteilt ist, sowie ein Verfahren zur lösungsmittelfreien Herstellung dieser wirkstoffhaltigen Schicht durch Mischen des wirksamen Bestandteils mit dem polymeren Trägermaterial in der Polymerschmelze bei Temperaturen von 170°C bis 200°C. Zur Fixierung des wirkstoffhaltigen Matrixfilms auf der Haut dient ein zusätzlicher Kontaktkleberfilm, der auf die Wirkstoffmatrix aufgebracht wird und, falls erforderlich, zusätzlich ein flächengrößeres Pflaster, das auf der von der Haut abgewandten Matrixseite auf den wirkstoffhaltigen Polymerfilm aufgebracht wird.

Ähnliche Aufbauprinzipien für transdermale Systeme oder Wirkstoffpflaster sind auch in **WO-A-93 23025** und **WO-A-95 09007** für Oxybutynin-haltige Pflasterzubereitungen beschrieben. Gemäß WO-A-95 09007 kann die Hautpenetration von Oxybutynin aus Polymermatrices durch Monoglyceride in Mischung mit Estern der Milchsäure erhöht werden, wobei diese Penetrationsenhancergemische keine oder nur geringe Hautreizungen erzeugen sollen.

Die in o.g. PCT-Dokumenten beschriebenen als Wirkstoffträger verwendeten Ethylen-Vinylacetat(EVA)-Copolymere werden zwecks Einarbeitung von Oxybutynin in einem geeigneten organischen Lösungsmittel aufgelöst oder durch Erwärmen geschmolzen. Die Erzeugung von Filmen erfolgt dann anschließend durch Beschichten und Entfernen des Lösungsmittels bzw. durch Kalandrieren der homogenen Polymer-/Wirkstoff-/Enhancermischung.

Zur Inkontinenzbehandlung sind gemäß **WO-A-93 23025** Oxybutynin-Plasmaspiegel in der Größenordnung von 0,5-2 ng/ml anzustreben, entsprechend einer Freisetzungsrate von 40-200 µg/h vorzugsweise 80-160 µg/h.

Nach **WO-A-96 33678** können diese Permeationsraten von Oxybutynin auch mit selbstklebenden monolithischen Systemen durch Einsatz von Triacetin als Penetrationsenhancer erreicht werden.

Von besonderem Interesse wegen ihres relativ guten Aufnahme- und Abgabevermögens für eine Vielzahl von Wirkstoffen sind in der Entwicklung von transdermalen Systemen Polymere auf Acrylsäureester und Methacrylsäureesterbasis. Um die Verwendung von Lösungsmitteln bei der Herstellung von Matrixsystemen auf Poly(meth)acrylatbasis zu vermeiden, ist in **DE-A-4310012** ein dermales therapeutisches System beschrieben, in dem eine oder mehrere Schichten aus Mischungen von Poly(meth)acrylaten aufgebaut und aus der Schmelze hergestellt werden und die erste Mischungskomponente aus (Meth)acrylpolymeren besteht, die funktionelle Gruppen enthalten, die zweite Mischungskomponente das Fließverhalten reguliert und nur unerhebliche Mengen an funktionellen Gruppen enthält. Die zusammengesetzten Systeme mit Poly(meth)acrylaten mit funktionellen Gruppen sollen eine gesteuerte Abgabe des oder der Wirkstoffe an bzw. durch die Haut und eine einfache Art der Herstellung ermöglichen. Ferner sollen die durch Kombination von Poly(meth)acrylaten mit niedriger Glastemperatur erhaltenen wirkstoffhaltigen Formulierungen Eigenschaften eines druckempfindlichen Hauthaftklebers aufweisen. Den Vorteilen in der Herstellung gegenüber lösungsmittelbasierten Verfahren stehen bei solchen Systemen jedoch erfahrungsgemäß eine Reihe von Nachteilen gegenüber, die bedingt sind durch:
1. Länger andauernde thermische Belastung aller TTS-Komponenten bei (1) der Herstellung der Polymerschmelze, (2) der homogenen Einarbeitung des oder der Wirkstoffe und/oder (3) der Beschichtung der heißen wirkstoffhaltigen Masse auf geeignete Trägermaterialien mit erhöhtem Risiko von Abbau- bzw. Zersetzungsreaktionen in der Polymerschmelze und/oder während der Lagerung der wirkstoffhaltigen Polymerfilme.
2. Schwierigkeiten in der Optimierung der Ko-/Adhäsionsbalance der poly(meth)acrylathaltigen Schicht für eine mehrtägige Anwendung, da eine Vernetzung des Acrylatcopolymerisates mittels kovalenter Bindungen während der Herstellung der wirkstoffhattigen Polymermatrix in der Schmelze nicht möglich ist, verbunden mit Problemen, die durch einen kalten Fluß der Polymermasse bei Anwendung auf der Haut und/oder bei Lagerung auftreten können.

Wie die vorgenannte Aufstellung zeigt, sind viele Pflasterkonstruktionen und hierfür verwendete Materialien bekannt. Gleichwohl besteht bis heute für viele in Transdermalen Therapeutischen Systemen verarbeitete Wirkstoffe ein großer Bedarf, TTS zur Verfügung zu stellen, die eine therapeutisch geforderte Wirkstoffabgabe ermöglichen, ohne dabei konstruktiv aufwendig zu sein und in der Gesamtschau ihrer Bestandteile eine optimale Beziehung darstellen. Dies gilt auch für den Wirkstoff Oxybutynin, wenn er transcutan verabreicht werden soll.

Therapeutisch wird Oxybutynin zur symptomatischen Behandlung der Hyperaktivität des Detrusors (Überfunktion des Harnblasenmuskels) mit häufigem Harndrang, vermehrtem nächtlichen Wasserlassen, zwingendem Harndrang, unfreiwilligem Harnabgang mit oder ohne Harndrang (Inkontinenz) eingesetzt. Die transcutane Applikation von Oxybutynin mittels eines TTS ist wünschenswert, da unter Umgehung des Magen-Darm-Traktes und der ersten Leberpassage Konzentrationsspitzen von Oxybutynin im Blut vermieden werden, die zum Auftreten unerwünschter Wirkungen wie Mundtrockenheit, Akkomodationsstörungen, Übelkeit und Schwindel führen können. Durch Umgehung des "first-pass"-Metabolismus in der Leber kann gegenüber der peroralen Gabe die Bioverfügbarkeit von Oxybutynin erhöht werden, bzw. die Gesamtdosis reduziert werden, die zum Erreichen der therapeutisch gewünschten Wirkung erforderlich sind.

Aufgabe der vorliegenden Erfindung ist es daher, die vorgenannten Nachteile der TTS mit Oxybutynin zu vermeiden und ein konstruktiv einfaches, hautverträgliches, über eine längere Lagerungs- und Applikationsdauer physikalisch und chemisch stabiles TTS zur transcutanen Verabreichung von Oxybutynin mit guten Hafteigenschaften zur Verfügung zu stellen, das
a) pro Flächeneinheit möglichst viel Wirkstoff an und durch die Haut freisetzt,
b) keinen Hautpenetrationsenhancer enthält,
c) lösemittelfrei ist und bei dem
d) die thermische Belastung des Wirkstoffes Oxybutynin im Herstellungsprozeß kurzzeitig erfolgt und möglichst niedrig ist.

Zur Lösung dieser Aufgabe wird ein TTS und ein Verfahren zu seiner Herstellung ohne Verwendung von Lösemitteln zur Verfügung gestellt, dessen besondere Zusammensetzung überraschenderweise der vorgenannten Aufgabenstellung genügt Es enthält eine Oxybutynin-haltige Matrixmasse in Form einer Schicht, die selbstklebend ist, wobei die Matrixmasse aus ammoniogruppenhaltigem (Meth)acrylatcopolymeren, mindestens einem Zitronensäuretriester und 5 - 25 % Gew.-% Oxybutynin besteht. Das TTS kann über mehrere Tage auf die Haut appliziert werden.

Bei dem erfindungsgemäßen TTS ist somit auf Grund seiner speziellen Zusammensetzung die Zugabe von Hautpenetrationsenhancem nicht erforderlich.

Im Sinne der Erfindung werden die nachfolgend genannten Begriffe und/oder Worte, wie folgt, verstanden:
- a) "lösemittelfrei":: zur Herstellung der Polymermatrices werden keine Lösungsmittel verwendet, die im Verlaufe des Herstellungsverfahrens wieder weitgehend entfernt werden, wie dieses im sogenannten "solvent based"-Verfahren geschieht.
- b) "mehrere Tage":: die TTS können zur therapeutischen Anwendung von 1 bis zu 7, vorzugsweise 1 bis 4 Tagen auf die Haut appliziert werden.
- c) "kurzfristige thermische Belastung des Wirkstoffes":: der in fester Form der bis zu 150°C erhitzten Polymermatrix zugegebene Wirkstoff wird innerhalb einer Minute in der Polymermatrix unter seinen Schmelzpunkt gekühlt.
- d) "feste Lösung":: der pharmazeutische Wirkstoff liegt im Polymeren/Zitronensäuretriester-Gemisch molekulardispers verteilt vor.

Nach einer weiteren erfindungsgemäßen Ausführung kann das vorbeschriebene TTS zusätzlich mit Ausnahme der Freisetzungsfläche seiner Oxybutynin-haltigen Matrix auf der Haut von einem größeren, jedoch wirkstofffreien Hautpflaster zur Fixierung an der Applikationsstelle umgeben sein.

Dieser Aufbau bedingt den Vorteil, daß den verschiedenen Hauttypen und Klimazonen Rechnung getragen werden kann. Weiterhin können einerseits die Ko-/Adhäsionseigenschaften des TTS, andererseits die Wirkstofflöslichkeit, Wirkstofflösungsgeschwindigkeit und das Freisetzungsverhalten weitgehend getrennt voneinander optimiert werden.

Nach einer Weiterbildung enthält die Matrixmasse vorzugsweise 10 - 20 Gew.-% Oxybutynin.

Schließlich kann die Oxybutynin-haltige Matrixmasse eine feste Lösung sein.

Die Bildung einer festen Lösung des Oxybutynins in dem ammoniogruppenhaltigen (Meth)acrylatpolymeren war nicht vorherzusehen und ist umso überraschender, als viele Wirkstoffe in Polymeren keine festen Lösungen (mit molekulardisperser Verteilung) bilden, sondern sich in Form fester Teilchen in das jeweilige Polymer einlagern, die elektronenmikroskopisch zu erkennen sind. Im Gegensatz zu festen Lösungen zeigen kristalline Wirkstoffe auch ein Debye-Scherrer-Diagramm.

Nach einer weiteren Ausführungsform der Erfindung enthält die Oxybutynin-haltige Matrixmasse vorzugsweise Zitronensäuretributylester.

Schließlich kann die Oxybutynin-haltige Matrixmasse eine Mischung aus Zitronensäuretributylester und Zitronensäuretriethylester enthalten.

Aufgrund der erfindungsgemäßen Zusammensetzung und des konstruktiven Aufbaus des TTS ist es überraschend, daß trotz hoher Wirkstoffkonzentrationen an Oxybutynin in der Polymermatrix eine ausreichende physikalische Stabilität des Systems bei Langzeitlagerung gewährleistet ist.

Für das als wirkstoffhaltige Polymermatrix verwendete Polymer war nicht zu erwarten, daß unmittelbar nach dem Aufkleben des TTS ein inniger Kontakt zwischen Wirkstoffmatrix und Haut hergestellt ist, der von der Qualität ist, daß ein über mehrere Tage selbständig klebendes TTS resultiert, das sowohl den therapeutischen als auch den gewerblichen, insbesondere den betriebswirtschaftlichen Erfordernissen, genügt.

Der Patientencompliance wird damit hervorragend Rechnung getragen.

Wenn man die Ausführungsform mit einem wirkstofffreien Hautpflaster/Overtape wählt, sind nur sehr kleinflächige Hautpflaster mit einem nur wenige mm Breite aufweisenden Kleberand erforderlich.

Dies ist sowohl wirtschaftlich als auch bezüglich der Patientencompliance von Vorteil.

Nach einer weiteren Ausführungsform der Erfindung weist die Trägerfolie des TTS matrixseitig eine Metalldampf- oder Oxidbeschichtung auf.

Das erfindungsgemäße TTS kann nach dem nachfolgend erläuterten Verfahren hergestellt werden.

Eine beschichtungsfähige Oxybutynin-haltige Matrixmasse wird durch Schmelzextrusion erzeugt, wobei der wirksame Bestandteil in eine bis zu 150°C heiße Polymerschmelze aus ammoniogruppenhaltigem (Meth)acrylatcopolymeren und Zitronensäuretriester kontinuierlich als Festsubstanz zudosiert wird, so daß eine Polymerschmelze mit einem Gehalt von bis zu 25 Gew.-% Oxybutynin und bis zu 33 Gew.-% Zitronensäuretriester erhalten wird und die heiße wirkstoffhaltige Polymerschmelze sofort nach erfolgter Zudosierung des Wirkstoffes kontinuierlich auf eine ablösbare Schutzschicht (= Substrat, Träger) in einer Dicke von 0,02 bis 0,4 mm beschichtet wird und das erhaltene 2-Schichtlaminat auf der anderen Matrixseite mit einer Deckschicht versehen wird.

Das mit einem zusätzlichen Hautpflaster oder Overtape versehene TTS wird, wie folgt, hergestellt.

Eine beschichtungsfähige Oxybutynin-haltige Matrixmasse wird durch Schmelzextrusion erzeugt, wobei der wirksame Bestandteil in eine bis zu 150°C heiße Polymerschmelze aus ammoniogruppenhaltigem (Meth)acrylatcopolymeren und Zitronensäuretriester kontinuierlich als Festsubstanz zudosiert wird, so daß eine Polymerschmelze mit einem Gehalt von bis zu 25 Gew.-% Oxybutynin und bis zu 33 Gew.-% Zitronensäuretriester erhalten wird und die heiße wirkstoffhaltige Polymerschmelze sofort nach erfolgter Zudosierung des Wirkstoffes kontinuierlich auf eine ablösbare Schutzschicht (= Substrat, Träger) in einer Dicke von 0,02 bis 0,4 mm beschichtet wird und das erhaltene 2-Schichtlaminat auf der anderen Matrixseite mit einer Deckschicht versehen wird und hierauf ein größeres wirkstofffreies Pflaster zur Fixierung des TTS auf der Haut aufgebracht wird.

Der wesentliche Vorteil des erfindungsgemäßen Verfahrens im Gegensatz zum sogenannten "batch-Verfahren" besteht darin, daß die den Wirkstoff enthaltende Polymermatrix (I) ohne Verwendung von organischen Lösungsmitteln hergestellt wird, und (II) die Zubereitung der wirkstoffhaltigen Matrixmasse und ihre weitere Verarbeitung zu einer wirkstoffhaltigen Schicht in einem kontinuierlichen und kosteneinsparenden Arbeitsgang erfolgen: Prozeßzeiten lassen sich auf wenige Minuten verkürzen. Die Gefahr von Zersetzungsreaktionen in der wirkstoffhaltigen Polymerschmelze kann hierdurch insoweit ausgeschlossen werden. Der Wirkstoff entspricht somit weiterhin den Qualitätskriterien des DAB und/oder der europäischen bzw. U.S. Pharmacopoe. Überraschend wurde dabei gefunden, daß die vollständige Auflösung und gleichmäßige Verteilung des Oxybutynins in der Polymerschmelze trotz der kurzen Prozeßzeiten unter den in den Beispielen weiter erläuterten Verfahrensbedingungen gewährleistet ist.

Femer werden durch die kontinuierliche Herstellung der Oxybutynin-haltigen Polymermasse Scaling-Up Probleme umgangen, d.h. bei Erhöhung der Ansatz- bzw. Chargengröße ist zur Herstellung der wirkstoffhaltigen Polymerschmelze und des Laminates kein Wechsel auf größere Produktionsanlagen erforderlich, der üblicherweise mit zeit- und kostenaufwendigen Installations-, Qualifizierungs- und Validierungsarbeiten sowie ggf. auch Rezepturänderungen verbunden ist.

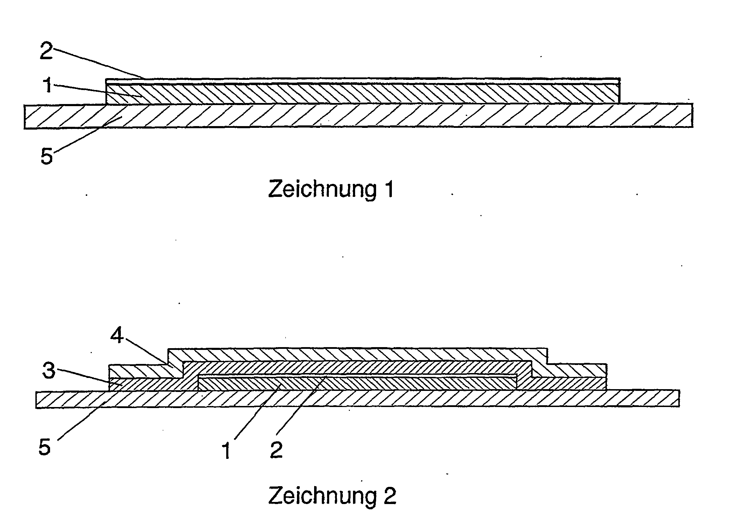

Der Aufbau der erfindungsgemäßen TTS ist in Zeichnungen 1 und 2 dargestellt.

Zeichnung 1 zeigt die Ausführungsform ohne Overtape, bestehend aus wirkstoffhaltiger Polymermatrix (1), ablösbarer Schutzfolie (5) und Deckfolie (2).

Zeichnung 2 zeigt die Ausführungsform mit Overtape. Zusätzlich zu den in der in Zeichnung 1 dargestellten Ausführungsform enthaltenen Schichten ist ein Overtape bestehend aus Trägerfolie (4) und Adhäsivfilm (3) enthalten.

Die Erfindung wird anhand folgender Beispiele erläutert:

### Beispiel 1:

Ein mit drei Dosiereinheiten ausgerüsteter Zweischneckenextruder wird kontinuierlich in aufeinanderfolgenden Verfahrenszonen mit Eudragit® RS 100 (Copolymerisat aus Ethylacrylat und Methylmethacrylat mit ca. 5% Trimethylammonioethylmethacrylatchlorid), Tributylcitrat und Oxybutynin beschickt, wobei die Mischung mit einem Gesamtdurchsatz von 5 kg /h bei einer Temperatur von 110-140 °C schmelzextrudiert wird. Aus der Dosiereinheit 1 wird Eudragit® RS 100 in einer Rate von 2,76 kg/h dem Verfahrensteil des Extruders zugeführt, aus Dosierstation 2 Tributylcitrat in einer Rate von 1,49 kg/h und schließlich aus Dosiereinheit 3 Oxybutynin in einer Rate von 0,75 kg/h. Nach dem Verlassen des Extruders wird die erhaltene heiße Oxybutynin-haltige Polymerschmelze über einen beheizten Zuführschlauch direkt in einem kontinuierlichen Strom dem Auftragskopf der Beschichtungsanlage zugeführt und mittels Schlitzdüse auf eine ca. 100 µm dicke silikonisierte Polyesterfolie (=Schutzfolie (5)) in einer Dicke von ca. 100 g pro m² aufgetragen (vgl. Zeichnung 1). Das Zweischichtlaminat wird nach Durchlaufen einer Walzenkühleinrichtung mit einer ca. 20 µm dicken Polyesterfolie (=Deckfolie (2)) abgedeckt.

Aus dem erhaltenen bahnförmigen Dreischichtlaminat werden anschließend 12 cm² große Stücke ausgestanzt.

### Beispiel 2:

Ein mit drei Dosiereinheiten ausgerüsteter Zweischneckenextruder wird kontinuierlich in aufeinanderfolgenden Verfahrenszonen mit Eudragit® RS 100 (Copolymerisat aus Ethylacrylat und Methylmethacrylat mit ca. 5% Trimethylammonioethylmethacrylatchlorid), Tributylcitrat und Oxybutynin beschickt, wobei die Mischung mit einem Gesamtdurchsatz von 5 kg /h bei einer Temperatur von 110-140 °C schmelzextrudiert wird. Aus der Dosiereinheit 1 wird Eudragit® RS 100 in einer Rate von 2,76 kg/h dem Verfahrensteil des Extruders zugeführt, aus Dosierstation 2 Tributylcitrat in einer Rate von 1,49 kg/h und schließlich aus Dosiereinheit 3 Oxybutynin in einer Rate von 0,75 kg/h. Nach dem Verlassen des Extruders wird die erhaltene heiße Oxybutynin-haltige Polymerschmelze über einen beheizten Zuführschlauch direkt in einen kontinuierlichen Strom dem Auftragskopf der Beschichtungsanlage zugeführt und mittels Schlitzdüse auf eine ca. 100 µm dicke silikonisierte Polyesterfolie (=Schutzfolie (5)) in einer Dicke von ca. 100 g pro m² aufgetragen (vgl. Zeichnung 2). Das Zweischichtlaminat wird nach Durchlaufen einer Walzenkühleinrichtung mit einer ca. 20 µm dicken Polyesterfolie (Deckfolie = (innere) Trägerfolie (2)) abgedeckt.

Aus dem erhaltenen bahnförmigen Dreischichtlaminat werden die Konturen 12 cm² großer Matrixstücke ausgestanzt, wobei die Deckfolie (2) und wirkstoffhaltige Polymermatrix (1), nicht aber die Schutzfolie (5) durchtrennt werden. Die entstehenden Zwischenstege werden abgegittert. Auf das erhaltene bahnförmige Laminat mit formatgestanzten TTS-Matrices wird eine 2-schichtig aufgebaute, selbstklebende Overtapefolie, bestehend aus einem ca. 80 *µ*m dicken Haftkleberfilm (3) auf Basis eines vernetzten Acrylatcopolymeren und einer (äußeren) Trägerfolie (4) aus Polyurethan, aufkaschiert. Das resultierende Laminat wird zu 20 cm² großen Pflastern, bestehend aus den Komponenten (1), (2), (3), (4), (5) entsprechend Zeichnung 2 ausgestanzt.

Der in den Beispielen 1 und 2 verwendete Zweischneckenextruder weist eine definierte Länge auf und besitzt als Dosiereinheit oder Dosierstation bezeichnete, räumlich auf der Längsachse des Extruders getrennte Zugabevorrichtungen für die einzusetzenden Stoffe. Weiterhin kann der Zweischneckenextruder über seine Länge in Verfahrenszonen eingeteilt werden, die unterschiedliche Bestimmungen erfüllen können. Beispielhaft sei die unterschiedliche thermische Einstellung einer Verfahrenszone im Verhältnis zu einer anderen genannt.

Fluxmessungen des Oxybutynins in vitro

### a) Fluxmessungen durch Mäusehaut

Ein TTS mit einer ausgestanzten Fläche von 2,5 cm² wird in einer horizontalen Diffusionszelle auf die Hornschichtseite der Bauch- und Rückenhaut haarloser Mäuse fixiert. Unmittelbar anschließend wird die Akzeptorkammer der Zelle mit auf 32°C vortemperierter Phospat-Pufferlösung pH 6,2 (Ph. Eur., pH 6,4 R ; mit Phosphorsäure auf pH 6,2 eingestellt) luftblasenfrei befüllt und das Freisetzungsmedium auf 32 ± 0,5 °C thermostatisiert.

Zu den Probeentnahmezeiten (nach 3, 6, 24, 30, 48, 54 und 72 Stunden) wird das Freisetzungsmedium gegen frisches, auf 32 ± 0,5 °C thermostatisiertes Medium ausgetauscht.

### b) Fluxmessungen durch Humanhaut

Die Prüfung erfolgte nach Tiemessen (Harry L.G.M. Thiemessen et al., Acta Pharm. Technol. 34(1998), 99 - 101). Nach der von diesem beschriebenen Methode erfolgte die Fluxmessung in einer Durchflußzelle an frisch präparierter, ca. 200 um dicker Humanhaut, die zur Akzeptorseite hin auf einer Silikonmembran aufliegt (Akzeptormedium : Phosphatpufferlösung pH 6,2; thermostatisiert auf 32 ± 0,5 °C).

Die Probeentnahmen erfolgten nach 3, 6, 9, 12, 15, 21, 24, 27, 30, 36, 42, 48, 54, 60, 66 und 72 Stunden.

Der Oxybutyningehalt in dem Freisetzungs- bzw. Akzeptormedium der beschriebenen Modelle wird mittels Hochdruckflüssigkeitschromatographie bestimmt. (Stationäre Phase: Supelcosil® LC-8-DB, 150 mm x 4,6 mm, 3 um; 45°C; Eluent: 29 Volumenteile Acetonitril und 71 Volumenteile einer Lösung von 8 g Triethanolamin in 1000 ml demineralisiertem Wasser, eingestellt auf pH 3,5 mit Phosphorsäure; UV Detektion bei 200 nm; Flußrate: 2,0 ml/min.; Injektionsvolumen: 25 µl).

Die Ergebnisse der Untersuchungen sind in Tabelle 1 für die Beispiele 1 und 2 dargestellt. Der Vergleich mit den aus dem Stand der Technik bekannten Fluxraten (vgl. WO-A-95 09007) von EVA-basierten Polymermatrixsystemen zeigt, daß das Oxybutynin aus den erfindungsgemäßen TTS entsprechend Beispielen 1 und 2 im "steady-state" in Raten durch die Haut freigesetzt wird, die gegenüber dem Stand der Technik in dessen oberen Bereich oder darüber liegen. Dies ist insofern überraschend, als die aus dem Stand der Technik bekannten Fluxraten von EVA-basierten Polymermatrixsystemen nur dadurch zu erzielen sind, indem spezielle Zusatzstoffe, die die Penetration von Oxybutynin durch die Humanhaut gegenüber gesättigten Lösungen beschleunigen, enthalten sind.

Wie die Ergebnisse der Tabelle 1 femer zeigen, wird mit Matrixsystemen gemäß der vorliegenden Erfindung überraschend eine mittlere Hautpermeationsrate (Flux) erreicht, die deutlich über den Sättigungsflüssen von Oxybutynin aus den in WO-A-93 23025 beschriebenen, niedrigviskosen Lösungen liegt, die ohne Hautpenetrationsenhancer (Substanzen, die eine Erhöhung der Hautdurchlässigkeit für Oxybutynin bewirken sollen) hergestellt wurden. Dabei wurde mit einem um 3°C niedriger temperierten Akzeptormedium als in den Literaturdaten beschrieben die Fluxmessung an den erfindungsgemäßen Pflastern sogar unter Bedingungen durchgeführt, welche für die Wirkstoffpenetration deutlich ungünstiger sind. Ferner zeigt Tabelle 1, daß mit Matrixsystemen gemäß Beispielen 1 und 2 über den Untersuchungszeitraum von 3 Tagen eine hohe Ausschöpfung der im polymeren Trägermaterial enthaltenen Wirkstoffgesamtmenge erreicht wird.

**Tabelle 1:**

| Oxybutynin-Fluxraten durch exzidierte Hautpräparationen (Beispiele 1 und 2) | | | | | |
|---|---|---|---|---|---|
| | Oxybutynin-gehalt von Matrix bzw. Donor (Gew.-%) | Steady-State Fluxrate (µg/cm²/h) | Mittlerer kumulativer Flux (mg/12 cm²) | | |
| | | | nach 24h | nach 48h | nach 72 h |
| Beispiel 1:Mäusehaut | 15 | 9,8 | 3,2 | 5,8 | 8,1 |
| n = 3 | (18mg/12 cm²) | | (17,5)* | (32,3)* | (45,0)* |
| Beispiel 1:Humanhaut | 15 | 8,9 | 1,6 | 4,2 | 6,7 |
| (Modell Thiemessen) | (18 mg/12 cm²) | | (8,9)* | (23,5)* | (37,1)* |
| n = 4 | | | | | |
| | | | | | |
| Literaturdaten ** | | | | | |
| | | | | | |
| (1) WO-A-95 09007 | 25 | Bereich von ca. 4 bis 10 | | | |
| | | | | | |
| (2) WO-A-93 23025: Oxybutyninlösungen ohne spezielle Penetrationsenhancer | gesättigte Lösungen | Bereich von ca. 2,5 bis 4 | | | |

| | | | | | |
|---|---|---|---|---|---|
| *) = kumulativer Flux in Gew.-% bezogen auf den Wirkstoffgehalt der Matrix (100% = 18 mg) | | | | | |
| **) = Prüfungen an exzidierter Humanhaut; Rezeptormedium 0,05 M Phospatpuffer; pH 6,5; 35 °C | | | | | |

## Patentansprüche

1. Transdermales therapeutisches System (TTS) zur transcutanen Verabreichung von Oxybutynin über mehrere Tage, **dadurch gekennzeichnet, daß** das TTS eine selbstklebende, schichtförmige Oxybutynin-haltige Matrixmasse enthält, die aus ammoniogruppenhaltigem (Meth)acrylatcopolymeren, mindestens einem Zitronensäuretriester und 5 -25 Gew.-% Oxybutynin besteht.

2. Transdermales therapeutisches System (TTS) zur transcutanen Verabreichung von Oxybutynin über mehrere Tage mit einer Fixierungshilfe für das TTS auf der Haut, **dadurch gekennzeichnet, daß** das TTS eine selbstklebende, schichtförmige Oxybutynin-haltige Matrixmasse enthält, die aus ammoniogruppenhaltigem (Meth)acrylatcopolymeren, mindestens einem Zitronensäuretriester und 5 - 25 Gew.-% Oxybutynin besteht, und dieses, mit Ausnahme seiner Freisetzungsfläche an der Applikationsstelle von einem größeren wirkstofffreien Pflaster zur Fixierung an der Haut umgeben ist.

3. TTS nach Ansprüchen 1 - 2, **dadurch gekennzeichnet, daß** die Oxybutynin-haltige Matrixmasse eine feste Lösung ist.

4. TTS nach Ansprüchen 1 - 3, **dadurch gekennzeichnet, daß** die Oxybutynin-haltige Matrixmasse Zitronensäuretributylester enthält

5. TTS nach Ansprüchen 1 - 4, **dadurch gekennzeichnet, daß** die Oxybutynin-haltige Matrixmasse Zitronensäuretributylester in Mischung mit Zitronensäuretriethylester enthält.

6. TTS nach Ansprüchen 1 - 5, **dadurch gekennzeichnet, daß** die Trägerfolie matrixseitig eine Metalldampf- oder Oxidbeschichtung aufweist.

7. Verfahren zur Herstellung eines Transdermalen Therapeutischen Systems (TTS) zur transcutanen Verabreichung von Oxybutynin, **dadurch gekennzeichnet, daß** einer bis zu 150°C erhitzten Polymerschmelze aus ammoniogruppenhaltigem (Meth)acrylatcopolymeren und Zitronensäuretriester kontinuierlich Oxybutynin als Festsubstanz zudosiert wird, so daß eine Polymerschmelze mit einem Gehalt von bis zu 25 Gew.-% Oxybutynin und bis zu 33 Gew.-% Zitronensäuretriester erhalten wird und die wirkstoffhaltige Polymerschmelze sofort nach erfolgter Zudosierung des Wirkstoffes kontinuierlich in einer Dicke von 0,02 - 0,4 mm auf einen Träger beschichtet wird und das erhaltene Laminat auf der anderen Matrixseite mit einer Deckschicht versehen wird.

8. Verfahren nach Anspruch 7, **dadurch gekennzeichnet, daß** einer bis zu 150°C erhitzten Polymerschmelze aus ammoniogruppenhaltigem (Meth)acrylatcopolymeren und Zitronensäuretriester kontinuierlich Oxybutynin als Festsubstanz zudosiert wird, so daß eine Polymerschmelze mit einem Gehalt von bis zu 25 Gew.-% Oxybutynin und bis zu 33 Gew.-% Zitronensäuretriester erhalten wird und die wirkstoffhaltige Polymerschmelze sofort nach erfolgter Zudosierung des Wirkstoffes kontinuierlich in einer Dicke von 0,02 - 0,4 mm auf einen Träger beschichtet wird, das erhaltene 2-Schichtlaminat mit einer Deckschicht versehen wird und hierauf ein größeres wirkstofffreies Pflaster zur Fixierung des TTS auf der Haut aufgebracht wird.

## Claims

1. Transdermal therapeutic system (TTS) for transcutaneous administration of oxybutynin over a plurality of days, **characterised in that** the TTS contains a self-adhesive oxybutynin-containing matrix body in layer form which comprises ammoniac-group-containing (meth)acrylate copolymers, at least one citric acid tri-ester and 5-25 % by weight of oxybutynin.

2. Transdermal therapeutic system (TTS) for transcutaneous administration of oxybutynin over a plurality of days, having an aid for fastening the TTS to the skin, **characterised in that** the TTS contains a self-adhesive oxybutynin-containing matrix body in layer form which comprises ammoniac-group-containing (meth)acrylate copolymers, at least one citric acid tri-ester and 5-25 % by weight of oxybutynin and the TTS, except for its release area at the point of application, is surrounded by a fairly large plaster free of active agent for fastening to the skin.

3. TTS according to claims 1-2, **characterised in that** the oxybutynin-containing matrix body is a solid solution.

4. TTS according to claims 1-3, **characterised in that** the oxybutynin-containing matrix body contains citric acid tributyl ester.

5. TTS according to claims 1-4, **characterised in that** the oxybutynin-containing matrix body contains citric acid tributyl ester mixed with citric acid triethyl ester.

6. TTS according to claims 1-5, **characterised in that** the substrate film has an oxide or vaporised metal coating in the matrix side.

7. Method of producing a transdermal therapeutic system (TTS) for transcutaneous administration of oxybutynin, **characterised in that** oxybutynin in the form of a solid substance is continuously metered into a polymer melt, heated to 150°C, of ammoniac-group-containing (meth)acrylate copolymers and citric acid tri-ester to produce a polymer melt having an oxybutynin content of up to 25% by weight and a citric acid tri-ester content of up to 33% by weight and immediately after the metering-in of the active agent the polymer melt containing the active agent is continuously coated onto a substrate to a thickness of 0.02-0.4 mm and the laminate obtained is provided with a covering layer on the other side of the matrix.

8. Method according to claim 7, **characterised in that** oxybutynin in the form of a solid substance is continuously metered into a polymer melt, heated to 150°C, of ammoniac-group-containing (meth)acrylate copolymers and citric acid triester to produce a polymer melt having an oxybutynin content of up to 25% by weight and a citric acid tri-ester content of up to 33% by weight and immediately after the metering-in of the active agent the polymer melt containing the active agent is continuously coated onto a substrate to a thickness of 0.02-0.4 mm, the two-layer laminate obtained is provided with a covering layer and a fairly large plaster free of active agent for fastening the TTS to the skin is applied thereto.

## Revendications

1. Système thérapeutique transdermique (STT) servant à l'administration transcutanée d'oxybutynine pendant plusieurs jours, ***caractérisé en ce que*** le STT contient une masse matricielle auto-adhésive, en forme de couche, contenant de l'oxybutynine, qui se compose d'un copolymère de (méth)acrylate contenant des groupes ammonio, d'au moins un triester d'acide citrique et de 5 à 25 % en poids d'oxybutynine.

2. Système thérapeutique transdermique (STT) servant à l'administration transcutanée d'oxybutynine pendant plusieurs jours avec un auxiliaire de fixation sur la peau, ***caractérisé en ce que*** le STT contient une masse matricielle auto-adhésive, en forme de couche, contenant de l'oxybutynine, qui se compose d'un copolymère de (méth)acrylate contenant des groupes ammonio, d'au moins un triester d'acide citrique et de 5 à 25 % en poids d'oxybutynine, et celui-ci est entouré, à l'exception de sa surface de libération sur le site d'application, d'un pansement plus grand dépourvu de substance active pour la fixation sur la peau.

3. STT selon les Revendications 1 et 2, ***caractérisé en ce que*** la masse matricielle contenant de l'oxybutynine est une solution solide.

4. STT selon les Revendications 1 à 3, ***caractérisé en ce que*** la masse matricielle contenant de l'oxybutynine contient du tributylester d'acide citrique.

5. STT selon les Revendications 1 à 4, ***caractérisé en ce que*** la masse matricielle contenant de l'oxybutynine contient du tributylester d'acide citrique en mélange avec du triéthylester d'acide citrique.

6. STT selon les Revendications 1 à 5, ***caractérisé en ce que*** la feuille support présente du côté de la matrice un revêtement de vapeur ou d'oxyde métallique.

7. Procédé de fabrication d'un système thérapeutique transdermique (STT) pour l'administration transcutanée d'oxybutynine, ***caractérisé en ce qu*'à** une masse fondue de polymères homogènes chauffée jusqu'à 150°C, composée de copolymères de (méth)acrylate contenant des groupes ammonio et de triester d'acide citrique, on ajoute de manière continue de l'oxybutynine sous forme de substance solide, de telle sorte qu'on obtienne une masse fondue de polymères avec une teneur allant jusqu'à 25 % en poids d'oxybutynine et jusqu'à 33 % en poids de triester d'acide citrique, et la masse fondue de polymères contenant la substance active est enduite de manière continue sur un substrat, juste après le dosage de la substance active, avec une épaisseur de 0,02 à 0,4 mm et le stratifié obtenu est muni d'une couche de couverture de l'autre côté de la matrice.

8. Procédé selon la Revendication 7, ***caractérisé en ce qu***'à une masse fondue de polymères homogènes chauffée jusqu'à 150°C, composée de copolymères de (méth)acrylate contenant des groupes ammonio et de triester d'acide citrique, on ajoute de manière continue de l'oxybutynine sous forme de substance solide, de telle sorte qu'on obtienne une masse fondue de polymères avec une teneur allant jusqu'à 25 % en poids d'oxybutynine et jusqu'à 33 % en poids de triester d'acide citrique, et la masse fondue de polymères contenant la substance active est enduite de manière continue sur un substrat, juste après le dosage de la substance active, avec une épaisseur de 0,02 à 0,4 mm, le stratifié à 2 couches obtenu est muni d'une couche de couverture et un pansement sans substance active, de plus grande surface, est appliqué par-dessus pour fixer le STT sur la peau.
